# EUROPEAN PATENT SPECIFICATION

(11) **EP 1 382 683 B1**
(45) Date of publication and mention of the grant of the patent: **22.02.2006**
(21) Application number: 03254408.2
(22) Date of filing: 11.07.2003
(51) Int. Cl.: C12P 7/26, C12N 15/53, C12N 9/02, C12R 1/19

(54) **Process for producing 3-hydroxycyclohexanone**
Verfahren zur Herstellung von 3-Hydroxycyclohexanon
Procédé de production de 3-hydroxycyclohexanone

(30) Priority: 15.07.2002 JP 2002205207
(43) Date of publication of application: 21.01.2004
(73) Proprietor: Sumitomo Chemical Company, Limited, Tokyo 104-8260 (JP)
(72) Inventor: Itoh, Nobuya, Toyama-shi, Toyama (JP); Wakita, Ryuhei, Toyonaka-shi, Osaka (JP)
(74) Representative: Cresswell, Thomas Anthony

(56) References cited:
- EP-A- 1 201 647
- EP-A- 1 213 354
- US-A- 4 455 373
- US-A- 5 233 095
- WEI Z-L ET AL: "Baker's yeast mediated mono-reduction of 1,3-cyclohexanediones bearing two identical C(2) substituents" TETRAHEDRON: ASYMMETRY, ELSEVIER SCIENCE PUBLISHERS, AMSTERDAM, NL, vol. 12, no. 2, 19 February 2001 (2001-02-19), pages 229-233, XP004230903 ISSN: 0957-4166
- WANG J-C ET AL: "Cloning, sequence analysis, and expression in Escherichia coli of the gene encoding phenylacetaldehyde reductase from styrene-assimilating Corynebacterium sp. strain ST-10" APPLIED MICROBIOLOGY AND BIOTECHNOLOGY, SPRINGER VERLAG, BERLIN, DE, vol. 52, no. 3, 1999, pages 386-392, XP002221908 ISSN: 0175-7598

### Sequence Listing

<110> Sumitomo Chemical Co., Ltd
   <120>
<130>
   <160> 15
<210> 1
   <211> 348
   <212> PRT
   <213> *Corynebacterium pseudodiphteriticum*
<210> 2
   <211> 1047
   <212> DNA
   <213> *Corynebacterium pseudodiphteriticum*
<220>
   <221> CDS
   <222> (1).. (1047)
<400> 2
   atg aag gcg atc cag tac acg aga atc ggc gcg gaa ccc gaa ctc acg    48
   Met Lys Ala Ile Gln Tyr Thr Arg Ile Gly Ala Glu Pro Glu Leu Thr
   1               5                   10                  15
   gag att ccc aaa ccc gag ccc ggt cca ggt gaa gtg ctc ctg gaa gtc    96
   Glu Ile Pro Lys Pro Glu Pro Gly Pro Gly Glu Val Leu Leu Glu Val
               20                  25                  30
   acc gct gct ggc gtc tgc cac tcg gac gac ttc atc atg agc ctg ccc   144
   Thr Ala Ala Gly Val Cys His Ser Asp Asp Phe Ile Met Ser Leu Pro
           35                  40                  45
   gaa gag cag tac acc tac ggc ctt ccg ctc acg ctc ggc cac gaa ggc   192
   Glu Glu Gln Tyr Thr Tyr Gly Leu Pro Leu Thr Leu Gly His Glu Gly
       50                  55                  60
   gca ggc aag gtc gcc gcc gtc ggc gag ggt gtc gaa ggt ctc gac atc   240
   Ala Gly Lys Val Ala Ala Val Gly Glu Gly Val Glu Gly Leu Asp Ile
   65                  70                  75                  80
   gga acc aat gtc gtc gtc tac ggg cct tgg ggt tgc ggc aac tgt tgg 288
   Gly Thr Asn Val Val Val Tyr Gly Pro Trp Gly Cys Gly Asn Cys Trp
   85 90 95
   cac tgc tca caa gga ctc gag aac tat tgc tct cgc gcc caa gaa ctc 336
   His Cys Ser Gln Gly Leu Glu Asn Tyr Cys Ser Arg Ala Gln Glu Leu
   100 105 110
   gga atc aat cct ccc ggt ctc ggt gca ccc ggc gcg ttg gcc gag ttc 384
   Gly Ile Asn Pro Pro Gly Leu Gly Ala Pro Gly Ala Leu Ala Glu Phe
   115 120 125
   atg atc gtc gat tct cct cgc cac ctt gtc ccg atc ggt gac ctc gac 432
   Met Ile Val Asp Ser Pro Arg His Leu Val Pro Ile Gly Asp Leu Asp
   130 135 140
   ccg gtc aag acg gtg ccg ctg acc gac gcc ggt ctg acg ccg tat cac 480
   Pro Val Lys Thr Val Pro Leu Thr Asp Ala Gly Leu Thr Pro Tyr His
   145 150 155 160
   gcg atc aag cgt tct ctg ccg aaa ctt cgc gga ggc tcg tac gcg gtt 528
   Ala Ile Lys Arg Ser Leu Pro Lys Leu Arg Gly Gly Ser Tyr Ala Val
   165 170 175
   gtc att ggt acc ggc ggt ctc ggc cac gtc gct att cag ctc ctc cgc 576
   Val Ile Gly Thr Gly Gly Leu Gly His Val Ala Ile Gln Leu Leu Arg
   180 185 190
   cac ctc tcg gcg gca acg gtc atc gct ttg gac gtg agc gcg gac aag 624
   His Leu Ser Ala Ala Thr Val Ile Ala Leu Asp Val Ser Ala Asp Lys
   195 200 205
   ctc gaa ctg gca acc aag gta ggc gct cac gaa gtg gtt ctg tcc gac 672
   Leu Glu Leu Ala Thr Lys Val Gly Ala His Glu Val Val Leu Ser Asp
   210 215 220
   aag gac gcg gcc gag aac gtc cgc aag atc act gga agt caa ggc gcc 720
   Lys Asp Ala Ala Glu Asn Val Arg Lys Ile Thr Gly Ser Gln Gly Ala
   225 230 235 240
   gca ttg gtt ctc gac ttc gtc ggc tac cag ccc acc atc gac acc gcg 768
   Ala Leu Val Leu Asp Phe Val Gly Tyr Gln Pro Thr Ile Asp Thr Ala
   245 250 255
   atg gct gtc gcc ggc gtc gga tca gac gtc acg atc gtc ggg atc ggg 816
   Met Ala Val Ala Gly Val Gly Ser Asp Val Thr Ile Val Gly Ile Gly
   260 265 270
   gac ggc cag gcc cac gcc aaa gtc ggg ttc ttc caa agt cct tac gag 864
   Asp Gly Gln Ala His Ala Lys Val Gly Phe Phe Gln Ser Pro Tyr Glu
   275 280 285
   gct tcg gtg aca gtc ccg tat tgg ggt gcc cgc aac gag ttg atc gaa 912
   Ala Ser Val Thr Val Pro Tyr Trp Gly Ala Arg Asn Glu Leu Ile Glu
   290 295 300
   ttg atc gac ctc gcc cac gcc ggc atc ttc gac atc tcg gtg gag acc 960
   Leu Ile Asp Leu Ala His Ala Gly Ile Phe Asp Ile Ser Val Glu Thr
   305 310 315 320
   ttc agt ctc gac aac ggt gcc gaa gcg tat cga cga ctg gct gcc gga 1008
   Phe Ser Leu Asp Asn Gly Ala Glu Ala Tyr Arg Arg Leu Ala Ala Gly
   325 330 335
   acg ctc agc ggc cgt gcg gtt gtg gtc cct ggt ctg tag 1047
   Thr Leu Ser Gly Arg Ala Val Val Val Pro Gly Leu
   340 345
<210> 3
   <211> 325
   <212> PRT
   <213> *Penicillium citrinum*
<400> 3
   Met Ser Asn Gly Lys Thr Phe Thr Leu Ser Asn Gly Val Lys Ile Pro 16
   1 5 10 15
   Gly Val Gly Phe Gly Thr Phe Ala Ser Glu Gly Ser Lys Gly Glu Thr 32
   20 25 30
   Tyr Thr Ala Val Thr Thr Ala Leu Lys Thr Gly Tyr Arg His Leu Asp 48
   35 40 45
   Cys Ala Trp Tyr Tyr Leu Asn Glu Gly Glu Val Gly Glu Gly Ile Arg 64
   50 55 60
   Asp Phe Leu Lys Glu Asn Pro Ser Val Lys Arg Glu Asp Ile Phe Val 80
   65 70 75 80
   Cys Thr Lys Val Trp Asn His Leu His Arg Tyr Glu Asp Val Leu Trp 96
   85 90 95
   Ser Ile Asp Asp Ser Leu Lys Arg Leu Gly Leu Asp Tyr Val Asp Met 112
   100 105 110
   Phe Leu Val His Trp Pro Ile Ala Ala Glu Lys Asn Gly Gln Gly Glu 128
   115 120 125
   Pro Lys Ile Gly Pro Asp Gly Lys Tyr Val Ile Leu Lys Asp Leu Thr 144
   130 135 140
   Glu Asn Pro Glu Pro Thr Trp Arg Ala Met Glu Lys Ile Tyr Glu Asp 160
   145 150 155 160
   Arg Lys Ala Arg Ser Ile Gly Val Ser Asn Trp Thr Ile Ala Asp Leu 176
   165 170 175
   Glu Lys Met Ser Lys Phe Ala Lys Val Met Pro His Ala Asn Gln Ile 192
   180 185 190
   Glu Ile His Pro Phe Leu Pro Asn Glu Glu Leu Val Gln Tyr Cys Phe 208
   195 200 205
   Ser Lys Asn Ile Met Pro Val Ala Tyr Ser Pro Leu Gly Ser Gln Asn 224
   210 215 220
   Gln Val Pro Thr Thr Gly Glu Arg Val Ser Glu Asn Lys Thr Leu Asn 240
   225 230 235 240
   Glu Ile Ala Glu Lys Gly Gly Asn Thr Leu Ala Gln Val Leu Ile Ala 256
   245 250 255
   Trp Gly Leu Arg Arg Gly Tyr Val Val Leu Pro Lys Ser Ser Asn Pro 272
   260 265 270
   Lys Arg Ile Glu Ser Asn Phe Lys Ser Ile Glu Leu Ser Asp Ala Asp 288
   275 280 285
   Phe Glu Ala Ile Asn Ala Val Ala Lys Gly Arg His Phe Arg Phe Val 304
   290 295 300
   Asn Met Lys Asp Thr Phe Gly Tyr Asp Val Trp Pro Glu Glu Thr Ala 320
   305 310 315 320
   Lys Asn Leu Ser Ala 325
   325
<210> 4
   <211> 978
   <212> DNA
   <213> *Penicillium citrinum*
<220>
   <221> CDS
   <222> (1)..(978)
<400> 4
   atg tct aac gga aag act ttc aca ttg agc aac ggc gtc aag att cct 48
   Met Ser Asn Gly Lys Thr Phe Thr Leu Ser Asn Gly Val Lys Ile Pro
   1 5 10 15
   ggc gtc ggc ttt ggt acc ttc gct agt gaa ggt tcc aag ggc gag acc 96
   Gly Val Gly Phe Gly Thr Phe Ala Ser Glu Gly Ser Lys Gly Glu Thr
   20 25 30
   tat act gct gtc acc act gcc ctg aag acc ggt tac cgt cac ttg gac 144
   Tyr Thr Ala Val Thr Thr Ala Leu Lys Thr Gly Tyr Arg His Leu Asp
   35 40 45
   tgt gcc tgg tac tac ctg aac gag ggt gag gtt ggt gag ggt atc cgt 192
   Cys Ala Trp Tyr Tyr Leu Asn Glu Gly Glu Val Gly Glu Gly Ile Arg
   50 55 60
   gac ttc ctg aag gag aac ccc tcg gtg aag cgt gag gac atc ttc gtc 240
   Asp Phe Leu Lys Glu Asn Pro Ser Val Lys Arg Glu Asp Ile Phe Val
   65 70 75 80
   tgc acc aag gtg tgg aac cac ctc cac cgt tat gag gac gtc ctc tgg 288
   Cys Thr Lys Val Trp Asn His Leu His Arg Tyr Glu Asp Val Leu Trp
   85 90 95
   tcc att gac gac tcc ctg aag cgt ctt gga ctt gac tac gtt gat atg 336
   Ser Ile Asp Asp Ser Leu Lys Arg Leu Gly Leu Asp Tyr Val Asp Met
   100 105 110
   ttc ctc gtt cac tgg ccc att gct gcc gag aag aat ggc cag ggt gag 384
   Phe Leu Val His Trp Pro Ile Ala Ala Glu Lys Asn Gly Gln Gly Glu
   115 120 125
   ccc aag att ggc cct gac ggc aaa tac gtc att ctc aag gac ctg acc 432
   Pro Lys Ile Gly Pro Asp Gly Lys Tyr Val Ile Leu Lys Asp Leu Thr
   130 135 140
   gag aac ccc gag ccc aca tgg cgc gct atg gag aag att tat gag gat 480
   Glu Asn Pro Glu Pro Thr Trp Arg Ala Met Glu Lys Ile Tyr Glu Asp
   145 150 155 160
   cgc aag gcc agg tcc att ggt gtc tcc aac tgg acc att gcc gac ctt 528
   Arg Lys Ala Arg Ser Ile Gly Val Ser Asn Trp Thr Ile Ala Asp Leu
   165 170 175
   gag aag atg tcc aag ttc gcc aag gtc atg cct cac gcc aac cag atc 576
   Glu Lys Met Ser Lys Phe Ala Lys Val Met Pro His Ala Asn Gln Ile
   180 185 190
   gag att cac ccc ttc ctg ccc aac gag gag ctg gtg cag tac tgc ttc 624
   Glu Ile His Pro Phe Leu Pro Asn Glu Glu Leu Val Gln Tyr Cys Phe
   195 200 205
   tcc aag aac att atg ccc gtg gcc tac tct cct ctg ggc tcg cag aac 672
   Ser Lys Asn Ile Met Pro Val Ala Tyr Ser Pro Leu Gly Ser Gln Asn
   210 215 220
   cag gtt ccc acc acc ggt gag cgg gtc agc gag aac aag act ctg aac 720
   Gln Val Pro Thr Thr Gly Glu Arg Val Ser Glu Asn Lys Thr Leu Asn
   225 230 235 240
   gag atc gcc gag aag ggc ggc aac acc ctt gct cag gtt ctt att gcc 768
   Glu Ile Ala Glu Lys Gly Gly Asn Thr Leu Ala Gln Val Leu Ile Ala
   245 250 255
   tgg ggt ctg cgc cgt ggc tac gtc gtt ctc ccc aag agc tcc aac ccc 816
   Trp Gly Leu Arg Arg Gly Tyr Val Val Leu Pro Lys Ser Ser Asn Pro
   260 265 270
   aag cgc att gag tcc aac ttc aag agc att gag ctc tcc gat gcc gac 864
   Lys Arg Ile Glu Ser Asn Phe Lys Ser Ile Glu Leu Ser Asp Ala Asp
   275 280 285
   ttt gaa gcc atc aat gcc gtt gcc aag ggt cgt cac ttc cgt ttc gtc 912
   Phe Glu Ala Ile Asn Ala Val Ala Lys Gly Arg His Phe Arg Phe Val
   290 295 300
   aac atg aag gat act ttc gga tat gat gtc tgg ccc gag gag acc gcc 960
   Asn Met Lys Asp Thr Phe Gly Tyr Asp Val Trp Pro Glu Glu Thr Ala
   305 310 315 320
   aag aac ctg tct gcg tga 978
   Lys Asn Leu Ser Ala
   325
<210> 5
   <211> 27
   <212> DNA
   <213> Artificial Sequence
<220>
   <223> Designed oligonucleotide primer for PCR
<400> 5
   gccatggcta tgaaggcgat ccagtac 27
<210> 6
   <211> 29
   <212> DNA
   <213> Artificial Sequence
<220>
   <223> Designed oligonucleotide primer for PCR
<400> 6
   cggatccgtc atcgaggcgt gcagctagc 29
<210> 7
   <211> 27
   <212> DNA
   <213> Artificial Sequence
<220>
   <223> Designed oligonucleotide primer for PCR
<400> 7
   gccatggcta tgtctaacgg aaagact 27
<210> 8
   <211> 29
   <212> DNA
   <213> Artificial Sequence
<220>
   <223> Designed oligonucleotide primer for PCR
<400> 8
   cggatccgtt ataatttcgt agagattca 29
<210> 9
   <211> 21
   <212> DNA
   <213> Artificial Sequence
<220>
   <223> Designed oligonucleotide primer for PCR
<400> 9
   gatcatcata gcaggagtca t 21
<210> 10
   <211> 21
   <212> DNA
   <213> Artificial Sequence
<220>
   <223> Designed oligonucleotide primer for PCR
<400> 10
   gaattcaaca ccagtcagct c 21
<210> 11
   <211> 786
   <212> DNA
   <213> Bacillus megaterium
<220>
   <221> CDS
   <222> (1).. (786)
<400> 11
   atg tat aaa gat tta gaa gga aaa gta gtt gtc ata aca ggt tca tct 48
   Met Tyr Lys Asp Leu Glu Gly Lys Val Val Val Ile Thr Gly Ser Ser
   1 5 10 15
   acc ggt tta gga aaa gca atg gcg att cgt ttt gcg aca gaa aaa gct 96
   Thr Gly Leu Gly Lys Ala Met Ala Ile Arg Phe Ala Thr Glu Lys Ala
   20 25 30
   aaa gta gtt gtg aac tat cgt tcg aaa gaa gaa gaa gct aac agc gtt 144
   Lys Val Val Val Asn Tyr Arg Ser Lys Glu Glu Glu Ala Asn Ser Val
   35 40 45
   tta gaa gaa att aaa aaa gtg ggc gga gag gct att gcc gtc aaa ggt 192
   Leu Glu Glu Ile Lys Lys Val Gly Gly Glu Ala Ile Ala Val Lys Gly
   50 55 60
   gat gta aca gtt gag tct gat gtg atc aat tta gtt caa tct gct att 240
   Asp Val Thr Val Glu Ser Asp Val Ile Asn Leu Val Gln Ser Ala Ile
   65 70 75 80
   aaa gaa ttt gga aag cta gac gtt atg att aat aac gca gga atg gaa 288
   Lys Glu Phe Gly Lys Leu Asp Val Met Ile Asn Asn Ala Gly Met Glu
   85 90 95
   aat ccg gtt tcg tct cat gaa atg tct tta agt gat tgg aat aaa gtc 336
   Asn Pro Val Ser Ser His Glu Met Ser Leu Ser Asp Trp Asn Lys Val
   100 105 110
   att gat acg aac tta acg gga gca ttt tta ggc agc cgt gaa gcg att 384
   Ile Asp Thr Asn Leu Thr Gly Ala Phe Leu Gly Ser Arg Glu Ala Ile
   115 120 125
   aaa tat ttt gtg gaa aat gat att aag gga aca gtt att aac atg tcg 432
   Lys Tyr Phe Val Glu Asn Asp Ile Lys Gly Thr Val Ile Asn Met Ser
   130 135 140
   agt gtt cac gag aaa att cct tgg cca tta ttt gtt cat tac gca gca 480
   Ser Val His Glu Lys Ile Pro Trp Pro Leu Phe Val His Tyr Ala Ala
   145 150 155 160
   agt aaa ggc gga atg aag ctc atg acc gaa aca ctt gca tta gaa tac 528
   Ser Lys Gly Gly Met Lys Leu Met Thr Glu Thr Leu Ala Leu Glu Tyr
   165 170 175
   gct cca aaa ggt att cgt gta aat aac att gga ccg gga gcg att aat 576
   Ala Pro Lys Gly Ile Arg Val Asn Asn Ile Gly Pro Gly Ala Ile Asn
   180 185 190
   aca ccg att aac gct gag aaa ttt gct gat cct gag cag cgt gca gat 624
   Thr Pro Ile Asn Ala Glu Lys Phe Ala Asp Pro Glu Gln Arg Ala Asp
   195 200 205
   gta gaa agc atg att cca atg gga tac att gga gag ccg gaa gaa att 672
   Val Glu Ser Met Ile Pro Met Gly Tyr Ile Gly Glu Pro Glu Glu Ile
   210 215 220
   gca gcg gtt gct gca tgg cta gct tct tca gag gca agt tat gta aca 720
   Ala Ala Val Ala Ala Trp Leu Ala Ser Ser Glu Ala Ser Tyr Val Thr
   225 230 235 240
   ggg att aca ctc ttt gct gac ggc ggt atg aca cag tac cca tca ttc 768
   Gly Ile Thr Leu Phe Ala Asp Gly Gly Met Thr Gln Tyr Pro Ser Phe
   245 250 255
   caa gca gga cgc gga taa 786
   Gln Ala Gly Arg Gly
   260
<210> 12
   <211> 261
   <212> PRT
   <213> *Bacillus megaterium*
<400> 12
   Met Tyr Lys Asp Leu Glu Gly Lys Val Val Val Ile Thr Gly Ser Ser    16
   1               5                   10                  15
   Thr Gly Leu Gly Lys Ala Met Ala Ile Arg Phe Ala Thr Glu Lys Ala    32
               20                  25                  30
   Lys Val Val Val Asn Tyr Arg Ser Lys Glu Glu Glu Ala Asn Ser Val    48
           35                  40                  45
   Leu Glu Glu Ile Lys Lys Val Gly Gly Glu Ala Ile Ala Val Lys Gly    64
       50                  55                  60
   Asp Val Thr Val Glu Ser Asp Val Ile Asn Leu Val Gln Ser Ala Ile    80
   65                  70                  75                  80
   Lys Glu Phe Gly Lys Leu Asp Val Met Ile Asn Asn Ala Gly Met Glu    96
                   85                  90                  95
   Asn Pro Val Ser Ser His Glu Met Ser Leu Ser Asp Trp Asn Lys Val   112
               100                 105                 110
   Ile Asp Thr Asn Leu Thr Gly Ala Phe Leu Gly Ser Arg Glu Ala Ile   128
   115 120 125
   Lys Tyr Phe Val Glu Asn Asp Ile Lys Gly Thr Val Ile Asn Met Ser 144
   130 135 140
   Ser Val His Glu Lys Ile Pro Trp Pro Leu Phe Val His Tyr Ala Ala 160
   145 150 155 160
   Ser Lys Gly Gly Met Lys Leu Met Thr Glu Thr Leu Ala Leu Glu Tyr 176
   165 170 175
   Ala Pro Lys Gly Ile Arg Val Asn Asn Ile Gly Pro Gly Ala Ile Asn 192
   180 185 190
   Thr Pro Ile Asn Ala Glu Lys Phe Ala Asp Pro Glu Gln Arg Ala Asp 208
   195 200 205
   Val Glu Ser Met Ile Pro Met Gly Tyr Ile Gly Glu Pro Glu Glu Ile 224
   210 215 220
   Ala Ala Val Ala Ala Trp Leu Ala Ser Ser Glu Ala Ser Tyr Val Thr 240
   225 230 235 240
   Gly Ile Thr Leu Phe Ala Asp Gly Gly Met Thr Gln Tyr Pro Ser Phe 256
   245 250 255
   Gln Ala Gly Arg Gly 261
   260
<210> 13
   <211> 27
   <212> DNA
   <213> Artificial Sequence
<220>
   <223> Designed oligonucleotide primer for PCR
<400>13
   gccatggcta tgtataaaga tttagaa 27
<210> 14
   <211> 23
   <212> DNA
   <213> Artificial Sequence
<220>
   <223> Designed oligonucleotide primer for PCR
<400> 14
   cggatccgtt atccgcgtcc tgc 23
<210> 15
   <211> 28
   <212> DNA
   <213> Artificial Sequence
<220>
   <223> Designed oligonucleotide primer for PCR
<400> 15
   cggatccgag cgcccaatac gcaaaccg 28

## Description

### BACKGROUND OF THE INVENTION

### Field of the Invention

This invention relates to a process for producing 3-hydroxycyclohexanone.

3-Hydroxycyclohexanone is a compound useful for producing substituted resorcinol derivatives described in JP-A 2001-294549, etc.

Wei *et al.,* Tetrahedron: Asymmetry, 2001, vol 12, pp229-233 discloses the Baker's yeast mediated mono-reduction of 1,3-cyclohexanediones bearing two identical C(2) substituents.

According to the present invention, there is provided a process for producing 3-hydroxycyclohexanone efficiently and readily by reacting 1,3-cyclohexanedione with an enzyme having an ability to reduce 1,3-cyclohexanedione to 3-hydroxycyclohexanone, a microorganism producing the enzyme, or a treated material of the microorganism, and recovering resulting 3-hydroxycyclohexanone.

That is, the present invention provides:
1. a process for producing optically active 3-hydroxycyclohexanone, which comprises the steps of:
   (a) reacting 1,3-cyclohexanedione with
      (i) an enzyme having an ability to reduce 1,3-cyclohexanedione to 3-hydroxycyclohexanone,
      (ii) a microorganism producing the enzyme defined in (i), or
      (iii) a treated material of the enzyme as defined in (i) or of the microorganism defined in (ii), and
   (b) recovering resulting 3-hydroxycyclohexanone; wherein
      i) said enzyme having the ability to reduce 1,3-cyclohexanedione to 3-hydroxycyclohexanone is an enzyme:
         derived from a microorganism belonging to the genus *Arthrobacter, Rhodotorula, Bacillus, Pseudomonas, Streptomyces, Candida, Corynebacterium* or *Penicillium;* or
         having any one of the following amino acid sequences: the amino acid sequence set forth in SEQ ID NO:1 or 3, an amino acid sequence comprising deletion, substitution, or addition of one or more amino acids in the amino acid sequence of SEQ ID NO:1 or 3, an amino acid sequence encoded by the nucleotide sequence set forth in SEQ ID NO:2 or 4, and an amino acid sequence encoded by a nucleotide sequence of a DNA having 90% or more sequence identity with DNA of SEQ ID NO: 2 or 4; or
      ii) said microorganism producing the enzyme is a transformant having a introduced plasmid containing:
         a nucleotide sequence of the nucleotide sequence of SEQ ID NO:2 or 4, or a nucleotide sequence of a DNA having 90% or more sequence identity with DNA of SEQ ID NO:2 or 4; or
      iii) said treated material of the microorganism producing the enzyme having an ability to reduce 1,3-cyclohexanedione to 3-hydroxycyclohexanone is dead microbial cells of the microorganism as defined in ii) above;
2. a process according to item 1, wherein the transformant is *Escherichia coli;*
3. a process according to item 1, wherein the enzyme has the amino acid sequence as set forth in SEQ ID NO:1;
4. a process according to item 1, wherein the enzyme has the amino acid sequence as set forth in SEQ ID NO:3;
5. a process according to item 1, wherein the enzyme has an amino acid sequence encoded by the nucleotide sequence as set forth in SEQ ID NO:2;
6. a process according to item 1, wherein the enzyme has an amino acid sequence encoded by the nucleotide sequence as set forth in SEQ ID NO:4;
7. a process according to item 1, wherein the transformant is a transformant produced by introducing:
   the plasmid containing
   a nucleotide sequence encoding the enzyme as defined in ii) and optionally
   a plasmid containing a nucleotide sequence that encodes an enzyme that regenerates a coenzyme on which the enzyme having the ability to reduce 1,3-cyclohexanedione to 3-hydroxycyclohexanone depends;
8. a process according to item 7, wherein the coenzyme is NADH/NAD⁺ (nicotinamide adenine dinucleotide) or NADPH/NADP⁺ (nicotinamide adenine dinucleotide phosphate);
9. a process according to item 1, wherein 1, 3-cyclohexanedione is allowed to react with the transformant or deadmicrobial cells thereof in the presence of a fatty alcohol.
10. a process according to item 9, wherein the fatty alcohol is an alcohol having a boiling point of 200°C or less.
11. a process according to item 9, wherein the fatty alcohol is 2-propanol.
12. a process according to item 1, wherein 1, 3-cyclohexanedione is allowed to react with the transformant or dead microbial cells thereof in the presence of glucose.
13. Optically active 3-hydroxycyclohexanone.

### DETAILED DESCRIPTION OF THE INVENTION

Hereinafter, this invention is described in more detail.

In this invention, "1,3-cyclohexanedione" is a compound of formula (2):

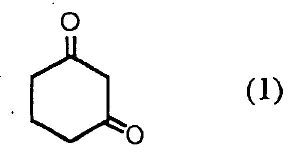

In this invention, "3-hydroxycyclohexanone" is a compound of formula (2):

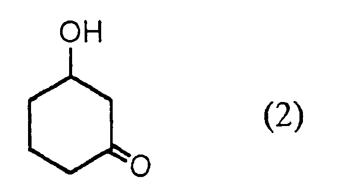

In this invention, "optically active 3-hydroxycyclohexanone" herein means a composition containing one optical isomer of 3-hydroxycyclohexanoneis based on the asymmetric carbon atom to which the hydroxyl group is bonded in formula (2) in an amount of more than 50% (dextrorotary compound and levorotary compound), or substantially pure optical isomer thereof, containing one optical isomer in an amount of, for example 95 to 100%, more preferably 98 to 100%.

The enzyme that may be used in the process of this invention is an enzyme having an ability to reduce 1,3-cyclohexanedione to form 3-hydroxycyclohexanone (preferably, optically active 3-hydroxycyclohexanone) (hereinafter, the enzyme is also referred to as the present reductase). By usual biochemical techniques or genetic engineering techniques, such an enzyme can be prepared from e.g. a microorganism producing the present reductase. When the microorganism producing the present reductase is, for example, a transformant, which includes a transformant produced by introducing at least one plasmid containing DNA of a nucleotide sequence encoding an amino acid sequence of an enzyme having an ability to reduce 1,3-cyclohexanedione to form 3-hydroxycyclohexanone (preferably, optically active 3-hydroxycyclohexanone) (hereinafter, the transformant is also referred to as the present transformant). When the microorganism producing the present reductase is a non-transformant (that is, a microorganism having the ability which was not artificially given but having such an ability inherently), the non-transformant includes a microorganism isolated through screening from commercial microorganisms or soil by using the above ability as an indicator, as described later in the Example. Examples of such microorganisms include microorganisms belonging to the genus *Arthrobacter, Rhodotorula, Bacillus, Pseudomonas, Streptomyces, Candida*, *Corynebacterium or Penicillium.* Further, the enzyme obtained in the process of this invention may be a commercial enzyme selected by screening with the above ability as an indicator. Examples of the enzyme that may be used include, for example, commercial enzymes such as KRED1001, KRED1002, KRED1003, KRED1004, KRED1005, KRED1006, KRED1007 and KRED1008, all of which are products of Biocatalytics.

On one hand, when the transformant that may be used in the process of the invention is a transformant, which is artificially endowed with an ability to reduce 1,3-cyclohexanedione to form 3-hydroxycyclohexanone (preferably, optically active 3-hydroxycyclohexanone) and an ability to regenerate a coenzyme on which the enzyme having the ability described above depends (hereinafter, the transformant is also referred to as the present transformant), the transformant is a transformant having one single introduced plasmid, or two introduced plasmids, which are:
one single plasmid containing a DNA consisting of a nucleotide sequence encoding an enzyme having the following two abilities (i) and (ii) as definedbelow, which can be artificially given, and
two plasmids, which are
a plasmid containing DNA consisting of a nucleotide sequence encoding an amino acid sequence of an enzyme having an ability (i) as defined below, which can be artificially given, and
a plasmid containing DNA consisting of a nucleotide sequence encoding an amino acid sequence of an enzyme having an ability (ii) as defined below, which can be artificially given, wherein said abilities are:
   (i) an ability to reduce 1,3-cyclohexanedione to form 3-hydroxycyclohexanone (preferably, optically active 3-hydroxycyclohexanone), and
   (ii) an ability to regenerate a coenzyme on which an enzyme having the ability (i) depends.

Such a transformant usually contains (1) an enzyme having two artificially given abilities, that is, an ability to reduce 1,3-cyclohexanedione to form 3-hydroxycyclohexanone (preferably, optically active 3-hydroxycyclohexanone) and an ability to regenerate a coenzyme on which an enzyme having the above ability depends or (2) two enzymes, that is, an enzyme having an ability to reduce 1,3-cyclohexanedione to form 3-hydroxycyclohexanone (preferably, optically active 3-hydroxycyclohexanone) and an enzyme having an ability to regenerate a coenzyme on which the above enzyme depends (hereinafter, the enzymes (1) and (2) is also referred to collectively as the present enzyme).

The "ability to reduce 1,3-cyclohexanedione to form 3-hydroxycyclohexanone (preferably, optically active 3-hydroxycyclohexanone)" is, for example, an ability possessed by an enzyme having an amino acid sequence selected from the group of the following amino acid sequences:
(a1) the amino acid sequence set forth in SEQ ID NO:1,
(a2) the amino acid sequence set forth in SEQ ID NO:3,
(b1) an amino acid sequence comprising the amino acid sequence of SEQ ID NO:1 having one or more deletion, substitution, or addition of amino acids and having an ability to reduce 1,3-cyclohexanedione to form 3-hydroxycyclohexanone (preferably, optically active 3-hydroxycyclohexanone),
(b2) an amino acid sequence comprising the amino acid sequence of SEQ ID NO:3 having deletion substitution, or addition of one or more amino acids, and having an ability to reduce 1,3-cyclohexanedione to form 3-hydroxycyclohexanone (preferably, optically active 3-hydroxycyclohexanone),
(c1) an amino acid sequence encoded by the nucleotide sequence set forth in SEQ ID NO:2,
(c2) an amino acid sequence encoded by the nucleotide sequence set forth in SEQ ID NO:4,
(d1) an amino acid sequence of an enzyme having an amino acid sequence encoded by a nucleotide sequence of DNA hybridizing under stringency conditions with DNA complementary to DNA consisting of the nucleotide sequence of SEQ ID NO: 2, said enzyme having the ability to reduce 1,3-cyclohexanedione to form 3-hydroxycyclohexanone (preferably, optically active 3-hydroxycyclohexanone),
(d2) an amino acid sequence of an enzyme having an amino acid sequence encoded by a nucleotide sequence of DNA hybridizing under stringency conditions with DNA complementary to DNA consisting of the nucleotide sequence of SEQ ID NO: 4, said enzyme having the ability to reduce 1,3-cyclohexanedione to form 3-hydroxycyclohexanone (preferably, optically active 3-hydroxycyclohexanone),
(e1) an amino acid sequence of an enzyme derived from a microorganism belonging to the genus *Corynebacterium,* said enzyme having an ability to reduce 1,3-cyclohexanedione to form 3-hydroxycyclohexanone (preferably, optically active 3-hydroxycyclohexanone), and
(e2) an amino acid sequence of an enzyme derived from a microorganism belonging to the genus *Penicillium*, said enzyme having an ability to reduce 1,3-cyclohexanedione to form 3-hydroxycyclohexanone (preferably, optically active 3-hydroxycyclohexanone).

The gene (also referred to hereinafter as the present reductase gene) having a nucleotide sequence encoding the amino acid sequence of the enzyme (the present reductase) having an ability to reduce 1,3-cyclohexanedione to form 3-hydroxycyclohexanone (preferably, optically active 3-hydroxycyclohexanone), which is used in preparing the present transformant, may be (1) a gene cloned from a naturally occurring gene, (2) a gene cloned from a naturally occurring gene, wherein a part of the nucleotide sequence of the cloned gene is deleted, substituted or added artificially (that is, by mutation (site-directed mutagenesis or mutation) of the naturally occurring gene), or (3) an artificially synthesized gene.

The "amino acid sequence having deletion, substitution, or addition of one or more amino acids" in (b), (b1) or (b2) above and the "amino acid sequence encoded by a nucleotide sequence of DNA hybridizing under stringency conditions with DNA complementary to DNA" in (d), (d1) or (d2) above encompass those with naturally occurring mutations by intracellular processing of an enzyme having the amino acid sequence of SEQ ID NO: 1 or 3, or with a species difference, individual difference and tissue difference of a living organism from which the enzyme was derived, or with artificial mutations of amino acids.

When the mutations by which "deletion, substitution, or addition (of amino acids) " (also referred to hereinafter as amino acid modifications) in (b), (b1) or (b2) are artificially performed, the techniques used include those in which DNA having a nucleotide sequence encoding the amino acid sequence of SEQ ID NO:1 or 3 is subjected to conventional site-specific mutation and then expressed in a usual manner. The site-specific mutation includes, for example, a method of using amber mutation (gapped duplex method, Nucleic Acids Res., 12, 9441-9456 (1984)) and a PCR method of using primers for mutation.

Amino acids thus mutated are at least 1 residue, specifically 1 to a few or more residues. The number of amino acids mutated may be in such a range that the ability to reduce 1,3-cyclohexanedione to form 3-hydroxycyclohexanone (preferably, optically active 3-hydroxycyclohexanone) can be found.

Among the modifications by deletion, substitution and addition, the modification of amino acids by substitution is particularly preferable. The substitution is more preferably substitution of amino acids by similar amino acids in properties such as hydrophobicity, charging, pK and stereostructure. Such substitution includes, for example, substitution among amino acids within the following group: <1> glycine and alanine, <2> valine, isoleucineandleucine, <3> aspartic acid, glutamicacid, asparagine and glutamine, <4> serine and threonine, <5> lysine and arginine, or <6> phenylalanine and tyrosine.

"Deletion, substitution or addition of amino acid(s)" in an amino acid sequence in the present invention typically means that there exists good sequence identity between an amino acid sequence and an amino acid sequence resulting therefrom by deletion, substitution, or addition of an amino acid(s), and sequence identity is typically, for example, 80% or more, preferably 90% or more and more preferably 95% or more sequence identity between the sequences.

As for two DNAs,"hybridizing under stringency conditions", typically means that there exists good sequence identity between their nucleotide sequences. For example, it typically means sequence identity of 80% or more, preferably 90% or more and more preferably 95% or more.

The "sequence identity" refers to identity between two DNAs or two proteins sequences. The "sequence identity" is determined by comparison between two sequences, as the subject of comparison, aligned in an optimum state. The DNAs or proteins as the subject of comparison may have undergone addition or deletion (e.g. gap etc.) in the optimum alignment between their two sequences. Such sequence identity can be calculated by making alignment by using e.g. Vector NTI and Clustal W algorism (Nucleic Acid Res., 22(22):4673-4680 (1994)). The sequence identity is typically determined by using sequence analysis software, specifically an analysis tool provided by Vector NTI, GENETYX-MAC or a public database. The public database is generally available in e.g. a homepage address http://www.ddbj.nig.ac.jp.

The hybridization used under "hybridizing under stringency conditions" in (d), (d1) or (d2) above can be carried out, for example, by methods described by Sambrook J., Frisch E. F., Maniatis T. in Molecular Cloning, 2nd ed., Cold Spring Harbor Laboratory Press, or by Southern hybridization described in "Cloning and Sequence" (in Japanese) supervised by Tadashi Watanabe and edited by Masahiro Sugiura, published in 1989 by Nohson Bunkasha. The "stringency conditions" refers, for example, to those conditions under which after a hybrid is formed at 65°C in 6×SSC (solution containing 900 mM NaCl, 90 mM trisodium citrate; in this case, a solution prepared by dissolving 175.3 g NaCl and 88.2 g trisodium citrate in 800 ml water, adjusting the pH with 10 N NaCl and adjusting the total volume to 1000 ml is referred to as 20×SSC), the hybrid is washed with 2×SSC at 50°C (Molecular Biology, 6.3.1-6.3.6., John Wiley & Sons, N.Y. (1989)). The salt concentration selected in the washing step can be, for example, under the conditions of 2×SSC at 50°C (low-stringency conditions) to the conditions of 0.1×SSC at 65°C (high-stringency conditions). The temperature in the washing step can be selected from e.g. room temperature (low-stringency condition) to 65°C (high-stringency condition). Further, both the salt concentration and temperature can also be changed.

The present reductase gene may be prepared according to e.g. the following preparation method.

According to usual genetic engineering techniques, chromosomal DNA is prepared from a microorganism belonging to the genus *Corynebacterium,* such as *Corynebacterium pseudodiphteriticum,* and PCR is carried out using the prepared chromosomal DNA as a template together with suitable primers, whereby DNA consisting of a nucleotide sequence encoding the amino acid sequence of SEQ ID NO: 1, DNA consisting of a nucleotide sequence encoding an amino acid sequence wherein one or more amino acids are deleted, substituted or added in the amino acid sequence of SEQ ID NO:1 or DNA having the nucleotide sequence of SEQ ID NO:2 is amplified to prepare the present reductase gene.

When PCR is carried out using an oligonucleotide having the nucleotide sequence of SEQ ID NO:5 and an oligonucleotide having the nucleotide sequence of SEQ ID NO:6 as primers together with the *Corynebacterium pseudodiphteriticum*-derived chromosomal DNA as a template, DNA consisting of the nucleotide sequence of SEQ ID NO:2 is amplified to prepare the present reductase gene.

The PCR conditions are those under which a reaction solution containing 20 µM each of 4 dNTPs, 15 pmol each of 2 oligonucleotide primers, 1.3 U Taq polymerase, and a cDNA library as a template is heated at 97°C (2 minutes) and then subjected to 10 cycles each consisting of reactions at 97°C (0.25 min)-50°C (0.5 min)-72°C (1.5 min) and then 20 cycles each consisting of reactions at 97°C (0.25 min)-55°C (0.5 min)-72°C (2.5 min), and the reaction solution is further kept at 72°C for 7 minutes.

A restriction enzyme recognition sequence etc. maybe added to the 5'-terminal of the primer used in PCR.

The DNA amplified in the manner described above is cloned in a vector by methods described by Sambrook J., Frisch E. F., Maniatis T. in Molecular Cloning, A Laboratory Manual, 2^{nd} ed., (1989), Cold Spring Harbor Laboratory Press, Current Protocols in Molecular Biology, (1987), John Wiley & Sons, Inc., ISBNO-471-50338-X, etc., whereby a recombinant vector can be obtained. The vector used includes, for example, pUC119 (Takara Shuzo Co., Ltd.), pTV118N (Takara Shuzo Co., Ltd.), pBluescriptII (Toyobo), pCR2.1-TOPO (Invitrogen), pTrc99A (Pharmacia), pKK223-3 (Pharmacia) etc. When the present reductase gene is prepared in an integrated state in the vector, it is conveniently used later in genetic engineering techniques.

By usual genetic engineering techniques (for example, methods described in "Shin Saibo Kogaku Jikken Protocol" (New Experimental Protocol in Cell Engineering), edited by Division of Oncology, the Institute of Medical Science, Tokyo University and published by Shujunsha (1993)), a cDNA library is prepared from a microorganism belonging to the genus Penicillium, such as Penicillium citrinum, and PCR is carried out using the prepared cDNA library as a template together with suitable primers, whereby DNA consisting of a nucleotide sequence encoding the amino acid sequence of SEQ ID NO: 3, DNA consisting of a nucleotide sequence encoding an amino acid sequence wherein one or more amino acids are deleted, substituted or added in the amino acid sequence of SEQ ID NO: 3 or DNA having the nucleotide sequence of SEQ ID NO:4 is amplified to prepare the present reductase gene.

When PCR is carried out using an oligonucleotide having the nucleotide sequence of SEQ ID NO:7 and an oligonucleotide having the nucleotide sequence of SEQ ID NO:8 as primers together with the Penicillium citrinum-derived cDNA library as a template, DNA consisting of the nucleotide sequence of SEQ ID NO:4 is amplified to prepare the present reductase gene.

When the enzyme having an ability to regenerate a coenzyme on which the enzyme having an ability to reduce 1,3-cyclohexanedione to form 3-hydroxycyclohexanone (preferably, optically active 3-hydroxycyclohexanone) depends (the gene for the enzyme is also referred to hereinafter as the present coenzyme regenerating enzyme gene) is an enzyme different from the present reductase enzyme, the present coenzyme regenerating enzyme gene may be prepared, for example, according to the following preparation method.

By genetic engineering techniques, chromosomal DNA is prepared from a microorganism belonging to the genus *Bacillus,* such as *Bacillus megaterium,* and PCR is carried out using the prepared chromosomal DNA as a template together with suitable primers, whereby DNA consisting of a nucleotide sequence encoding the amino acid sequence of SEQ ID NO:12, DNA consisting of a nucleotide sequence encoding an amino acid sequence wherein one or more amino acids are deleted, substituted or added in the amino acid sequence of SEQ ID NO:12 or DNA having the nucleotide sequence of SEQ ID NO:11 is amplified to prepare the present coenzyme-regenerating enzyme gene.

When PCR is carried out using an oligonucleotide having the nucleotide sequence of SEQ ID NO: 9 and an oligonucleotide having the nucleotide sequence of SEQ ID NO: 10 as primers together with the *Bacillus megaterium*-derived chromosomal DNA as a template, DNA consisting of the nucleotide sequence of SEQ ID NO:11 is amplified to prepare the present coenzyme regenerating enzyme gene.

The PCR conditions are for example those under which a reaction solution containing 20 µM each of 4 dNTPs, 15 pmol each of 2 oligonucleotide primers, 1.3 U Taq polymerase, and a cDNA library as a template is heated at 97°C (2 minutes) and then subjected to 10 cycles each consisting of reactions at 97°C (0.25 min)-50°C (0.5 min)-72°C (1.5 min) and then 20 cycles each consisting of reactions at 97°C (0.25 min)-55°C (0.5 min)-72°C (2.5 min), and the reaction solution is further kept at 72°C for 7 minutes.

A restriction enzyme recognition sequence etc. may be added to the 5'-terminal of the primer used in PCR.

The DNA amplified in the manner described above is cloned in a vector by methods described by Sambrook J., Frisch E. F., Maniatis T. in Molecular Cloning, A Laboratory Manual, 2^{nd} ed., (1989), Cold Spring Harbor Laboratory Press, Current Protocols in Molecular Biology, (1987), John Wiley & Sons, Inc., ISBNO-471-50338-X, etc., whereby a recombinant vector can be obtained. The vector used includes, for example, pUC119 (Takara Shuzo Co., Ltd.), pTV118N (TakaraShuzoCo., Ltd.), pBluescriptII (Toyobo), pCR2.1-TOPO (Invitrogen), pTrc99A (Pharmacia), pKK223-3 (Pharmacia) etc. When the present reductase gene is prepared in an integrated state in the vector, it is conveniently used later in genetic engineering techniques.

The method of preparing the present transformant includes, for example, (1) a method of preparing a recombinant plasmid capable of expressing the present reductase gene in host cells, such as DNA wherein the present reductase gene and a promoter capable of functioning in host cells are linked in an operable form (for example, a recombinant plasmid constructed by ligating a region involved in expression regulation, such as a promoter and a terminator, with the present reductase gene, or a recombinant plasmid expressing the gene as an operon containing a plurality of cistrons, such as lactose operon) and then introducing it into host cells, (2) a method of preparing a single recombinant plasmid capable of expressing the gene in host cells, such as DNA wherein the two genes, that is, the reductase gene and the coenzyme regenerating enzyme gene, and a promoter capable of functioning in host cells are ligated in an operable form, and then introducing it into host cells, and (3) a method of preparing two recombinant plasmids capable expressing the two genes respectively, such as DNAs wherein one of the two genes, that is, the reductase gene or the coenzyme regenerating enzyme gene, and a promoter capable of functioning in host cells are ligated in an operable form, and then introducing the two plasmids having the reductase gene and the coenzyme regenerating enzyme gene respectively into host cells. Further, a method of introducing either or both of the genes into the host cell chromosome can also be utilized.

The method of producing the transformant by introducing the single recombinant plasmid into host cells includes, for example, a method of constructing the recombinant plasmid by ligating e.g. regions involved in expression regulation, such as a promoter and a terminator, with both the genes and a method of constructing the recombinant plasmid expressing the gene as operon containing a plurality of cistrons, such as lactose operon.

Preferably, the recombinant plasmid is, for example, a plasmid which contains genetic information replicable in host cells, is capable of autonomous multiplication, can be easily isolated and purified from the host cells, and has the gene encoding the present reductase introduced in an operable form into an expression vector having a detectable marker and a promoter operable in the host cells. As the expression vector, various kinds of vectors are commercially available.

The terms "operable form" mean that upon transformation of the recombinant plasmid into host cells, the present reductase gene is linked with a promoter so as to be expressed under the control of the promoter. The promoter includes a lactose operon promoter from *E. coli*, tryptophan operon promoter from *E. coli*, and synthetic promoters capable of functioning in *E. coli,* such as tac promoter and trc promoter. A promoter regulating the expression of the present reductase gene in *Corynebacterium pseudodiphteriticum, Penicillium citrinum* or *Bacillus* megaterium may also be utilized.

When the expression vector used is a vector containing a selective marker gene (for example, an antibiotic resistance gene such as kanamycin resistance gene or neomycin resistance gene), the transformant into which the vector was introduced can be selected easily by using e.g. the phenotype of the selective marker gene as an indicator.

If there is need for induction of higher expression, a ribosome-binding region may be ligated with a region upstream of the gene having the nucleotide sequence encoding the amino acid sequence of the present reductase. The ribosome-binding region used include those described by Guarente L. et al. (Cell 20, p. 543) and Taniguchi et al. (Genetics of Industrial *Microorganisms*. *p. 202*, Kodansha).

The host cells include microbial cells such as procaryotes (for example, the genera *Escherichia*, *Bacillus*, *Corynebacterium*, *Staphylococcus* and *Streptomyces*) and eucaryotes (for example, the genera *Saccharomyces, Kluyveromyces* and *Aspergillus*), insect cells and mammalian cells. A preferable example of host cells is *Escherichia coli* from the viewpoint of easiness of preparation of a large amount of the transformant.

When the plasmid capable of expressing the present reductase in host cells is to be introduced into the host cells, a conventional method may be used depending on the host cells used, and the method include, for example, the calcium chloride method described in Molecular Cloning: A Laboratory Manual, 2nd ed., (1989), Cold Spring Harbor Laboratory Press, Current Protocols in Molecular Biology, (1987), John Wiley & Sons, Inc., ISBNO-471-50338-X, etc., and the electroporation method described in Methods in Electroporation: Gene Pulser/E. coli Pulser System, Bio-Rad Laboratories, (*1993*), etc.

The transformant harboring the plasmid capable of expressing the reductase gene and/or the coenzyme regenerating gene in host cells can be selected from the host cells by using the phenotype, as an indicator, of the selective marker gene contained in the vector, as described above.

Whether or not the host cells into which the plasmid was introduced (that is, the transformant) carry the reductase gene and/or the coenzyme regenerating enzyme gene can be confirmed by examining a restriction enzyme site or analyzing the nucleotide sequence or by Southern hybridization, Western hybridization, etc. according to conventional methods described in Molecular Cloning: A Laboratory Manual, 2^{nd} ed., (1989), Cold Spring Harbor Laboratory Press, etc.

The present transformant can be cultured by a conventional method used in culture of microorganisms, insect cells or mammalian cells. For example, *Escherichia coli* is cultured in a medium containing a suitable carbon and nitrogen sources and trace nutrients such as vitamins. The culture method may be either culture on solid or culture in liquid such as test-tube shake culture, reciprocating shake culture, Jar Fermenter culture or tank culture, preferably culture in liquid such as aeration shake culture.

Although the culture temperature can be changed in the range where the transformant can grow, the temperature is usually about 10 to 50°C, preferably about 20 to 40°C.

The pH value of the medium is preferably in the range of about 6 to 8.

The culture time is usually about 1 day to about 5 days, depending on culture conditions.

As the medium for culturing the present transformant, various mediums containing a carbon source, a nitrogen source, organic salts and inorganic salts used ordinarily in culturing host cells such as microorganisms can be used.

The carbon source includes, for example, glucose, dextrin, sugars such as sucrose, sugar alcohols such as glycerol, organic acids such as fumaric acid, citric acid and pyruvic acid, animal oils, vegetable oils and molasses. The amount of these carbon sources added to the medium is usually about 0.1 to 30% (w/v) relative to the culture solution.

The nitrogen source includes, for example, naturally occurring nitrogen sources such as meat extract, peptone, yeast extract, malt extract, soybean powder, corn steep liquor, cottonseed powder, dried yeast and casamino acid, amino acids, inorganic acid ammonium salts such as sodium nitrate, inorganic acid ammonium salts such as ammonium chloride, ammonium sulfate and ammonium phosphate, organic acid ammonium salts such as ammonium fumarate and ammonium citrate, and urea. Among these, the organic acid ammonium salts, naturally occurring organic nitrogen sources and amino acids can also be used as the carbon source. The amount of these nitrogen sources to the medium is usually about 0.1 to 30% (w/v) based on the culture solution.

The organic salts and inorganic salts include, for example, chlorides, sulfates, acetates, carbonates and phosphates such as those of potassium, sodium, magnesium, iron, manganese, cobalt and zinc. Specific examples include sodium chloride, potassium chloride, magnesium sulfate, ferrous sulfate, manganese sulfate, cobalt chloride, zinc sulfate, copper sulfate, sodium acetate, calcium carbonate, monopotassium hydrogen phosphate and dipotassium hydrogen phosphate. The amount of these organic acid salts and/or inorganic acid salts to the medium is usually about 0.0001 to 5% (w/v) based on the culture solution.

For the transformant produced for example by introducing DNA wherein a promoter induced by allolactose, such as tac promoter, trc promoter or lac promoter, and the gene having the nucleotide sequence encoding the amino acid sequence of the present enzyme were linked in an operable form, a small amount of isopropyl thio-β-D-galactoside (IPTG) can also be added to the medium, as an inducer for inducing production of the present enzyme.

The present transformant can be recovered in the form of a pellet by centrifuging its culture obtained by culturing. Before recovery, the transformant may be washed if necessary with a buffer such as 100 mM monopotassium phosphate-dipotassium phosphate buffer (pH 6.5).

In the process of this invention, the enzyme having an ability to reduce 1,3-cyclohexanedione, a microorganism producing the enzyme or a treated material thereof is used as the catalyst for reducing 1,3-cyclohexanedione to form 3-hydroxycyclohexanone (preferably, optically active 3-hydroxycyclohexanone).

When the microorganism producing the enzyme having an ability to reduce 1,3-cyclohexanedione is used in the reaction in the process of this invention, the microorganism is used in the form of (1) the culture solution and (2) the wet microorganism obtained for example by centrifuging the culture solution to collect the microorganism and then washing the microorganism with a buffer or water.

When the enzyme having an ability to reduce 1,3-cyclohexanedione or the treated material derived from the microorganism producing the enzyme is used in the reaction in the process of this invention, the enzyme or the treated material used is obtained by subjecting the wet microorganism obtained for example by centrifuging the culture solution to collect the microorganism and then washing the microorganism with a buffer or water to (1) treatment with an organic solvent (acetone, ethanol etc.), (2) lyophilization, (3) alkali treatment, and (4) physical or enzymatic disruption, or the resulting treated material may be further immobilized in a known method.

Among the enzyme having an ability to reduce 1,3-cyclohexanedione, the microorganism producing the enzyme and the treated material thereof, the transformant artificially endowed with an ability to reduce 1,3-cyclohexanedione or its dead cells are preferably used.

From the present transformant, its dead cells can be prepared in the following method.

The method of preparing the dead cells includes, for example, a physical cell killing method (heating, drying, freezing, light rays, sonication, filtration, electrification) and a killing method using chemicals (alkali, acid, halogen, oxidizing agent, sulfur, boron, arsenic, metal, alcohol, phenol, amine, sulfide, ether, aldehyde, ketone, cyan, antibiotic). Preferably, a treatment method with minimum inactivation of the activity of the present reductase and with less contamination and pollution in the reaction system is suitably selected from these killing methods , depending on various reaction conditions.

The transformant or its dead cells thus prepared may be used for example in the form of lyophilized cells, cells treated with an organic solvent, or dried cells, or in an immobilized form (immobilized product).

The method of obtaining the immobilized product includes, for example, a method of binding to carriers (method of adsorbing the present transformant or its dead cells onto inorganic carriers such as silica gel and ceramics or onto cellulose and ion-exchange resin) and an inclusion method (method of entrapping the present transformant or its dead cells into networks in polymers such as polyacrylamide, sulfur-containing polysaccharide gel (e.g. carrageenan gel), alginic acid gel and agar gel).

The reaction of the process of this invention is described.

In the process of the invention, the reaction for converting 1,3-cyclohexanedione into 3-hydroxycyclohexanone (preferably, optically active 3-hydroxycyclohexanone) is achieved by allowing the present reductase, a microorganism producing the enzyme or a treated material thereof (and a coenzyme if necessary) to react with 1,3-cyclohexanedione.

The reaction is carried out usually in the presence of water. Water may be in the form of a buffer, and the buffer used in this case includes, for example, alkali metal phosphates such as sodium phosphate and potassium phosphate and alkali metal salts such as sodium acetate and potassium acetate.

When the buffer is used as a solvent, its amount is usually 1 to 300 parts by weight, preferably 5 to 100 parts by weight, relative to 1 part by weight of 1,3-cyclohexanedione.

In the reaction, 1,3-cyclohexanedione may be added continuously or successively to the reaction system.

The reaction temperature is usually about 0 to 70°C, preferably about 10 to 40°C, from the viewpoint of stability and reaction rate of the present reductase, or the present reductase contained in a microorganism producing the enzyme or its treated material.

Although the reaction pH may be suitably changed within such a range that the reaction proceeds, the pH is, for example, 5 to 8.

The reaction can also be conducted in the coexistence of an organic solvent besides water. The organic solvent in this case includes, for example, ethers such as tetrahydrofuran, t-butyl methyl ether and isopropyl ether, hydrocarbons such as toluene, hexane, cyclohexane, heptane, isooctane and decane, alcohols such as t-butanol, methanol, ethanol, isopropanol and n-butanol, sulfoxides such as dimethyl sulfoxide, ketones such as acetone, nitriles such as acetonitrile, and mixtures thereof.

The amount of the organic solvent used in the reaction is usually not higher than 100 parts by weight, preferably not higher than 70 parts by weight, based on 1,3-cyclohexanedione.

The reaction is conducted preferably in the presence of a coenzyme such as NADH or NADPH.

The amount of the coenzyme used in the reaction is usually not higher than 0.5 part by weight, preferably not higher than 0.1 part by weight, based on 1,3-cyclohexanedione.

In the process of this invention, it is preferable to use the present coenzyme-regenerating enzyme having an ability to regenerate a coenzyme on which the enzyme having an ability to reduce 1,3-cyclohexanedione to form 3-hydroxycyclohexanone (preferably, optically active 3-hydroxycyclohexanone) depends, that is, the coenzyme-regenerating enzyme having an ability to convert an oxidized coenzyme (electron acceptor), which is generated from a reduced coenzyme (electron donor) in a stoichiometric amount in an elementary reaction for reducing 1,3-cyclohexanedione, into a reduced coenzyme (electron donor) again. In this case, the present coenzyme-regenerating enzyme may be an independent enzyme from the present reductase catalyzing the reduction reaction, or it may be an enzyme that has an ability to catalyze the reduction reaction, or both may be used together.

As to whether the present reductase is an enzyme that has an ability to regenerate the coenzyme together can be confirmed, for example, by contacting the isolated present enzyme with an electron-donating compound in the presence of an oxidized coenzyme and detecting a reduced coenzyme (electron donor).

Examples of the present coenzyme-regenerating enzyme includes, for example, glucose dehydrogenase, alcohol dehydrogenase, aldehyde dehydrogenase, amino acid dehydrogenase and organic dehydrogenase (malate dehydrogenase etc.) etc. Particularly, the coenzyme-regenerating enzyme is preferably an enzyme regenerating a coenzyme by oxidizing a fatty alcohol, as an electron-donating compound, (for example, alcohols having a boiling point of 200°C or less, such as 2-propanol, 2-butanol, 2-pentanol, 2-hexanol, 2-heptanol and 2-octanol). The molar ratio of the fatty alcohol to 1,3-cyclohexanedione is not higher than 10, preferably not higher than 10.

The reaction can also be carried out by adding sugars such as glucose, sucrose, fructose or the like, alcohols such as ethanol or the like, and a surfactant, if necessary.

The progress of the reaction can be monitored, for example, in terms of the amount of the starting compound in the reaction solution by liquid chromatography, gas chromatography etc. The reaction time is usually in the range of 5 minutes to 10 days, preferably 30 minutes to 4 days.

After completion of the reaction, the desired product may be collected by a recovery method conventionally used in the process for producing compounds by using an enzyme or a microorganism as a catalyst. For example, the reaction solution is extracted with an organic solvent such as hexane, heptane, tert-butyl methyl ether, ethyl acetate or toluene. If necessary, the reaction solution may be filtered or centrifuged to remove insolubles before the extraction is conducted. Then, the extracted organic layer can be dried to recover the desired product as a concentrate. The desired product can further be purified by column chromatography, if necessary.

### EXAMPLES

Hereinafter, this invention is described in more detail by reference to production examples etc., but this invention is not limited to the examples.

### Example 1 (Preparation of the present reductase gene (No. 1) and preparation of a transformant containing the present reductase gene)

Plasmid pKAR containing DNA consisting of the nucleotide sequence of SEQ ID NO:2 was prepared in the following manner.

First, a DNA fragment containing the DNA shown in SEQ ID NO: 2 was excised with PstI and SmaI from known plasmid pUAR (The plasmid was deposited under the Budapest Treaty as FERM BP-7752, which had been originally deposited as FERM P-18127. The depositary institution was International Patent Organism Depositary(IPOD), formerly known as the National Institute of Bioscience and Human-Technology(NIBH)) described in Appl Microbial Biotechnol 52:386-392 (1999). The excised DNA fragment was inserted into a region downstream of a Tac promoter in PstI/SmaI-treated pKK223-3 vector (Amersham Pharmacia Biotech). Plasmid pKAR was constructed in this manner.

The constructed plasmid pKAR was used to transform E. coli JM109.

Then, a liquid medium (solution of 10 g trypton, 5 g yeast extract and 5 g sodium chloride in 1000 ml water, adjusted to pH 7.0 by dropping 1 N aqueous sodium hydroxide), 100 ml, was introduced into a flask and sterilized, and 100 µg/ml ampicillin, 0.01% (w/v) ZnCl₂ and 0.4 mM isopropyl thio-β-D-galactoside (IPTG) were added thereto. Into the medium thus prepared was inoculated 0.3 ml culture of the transformant obtained above (E. coli JM109/pKAR) previously cultured in a liquid medium having the above composition, and the transformant was cultured under shaking at 30°C for 14 hours.

After the culture, the resulting culture was centrifuged (15000×g, 15 minutes, 4°C) to recover the microorganism. The recovered microorganism was suspended in 30 ml of 50 mM monopotassium phosphate-dipotassium phosphate buffer (pH 7.0), and this suspension was centrifuged (15000×g, 15 minutes, 4°C), whereby the washed microorganism i.e. the transformant harboring the present reductase gene was obtained.

### Example 2 (Preparation of the present enzyme gene (No. 2))

### (2-1) Preparation of chromosomal DNA

A liquidmedium (solution of 5 g trypton, 2.5 g yeast extract, 4 g sodium chloride, 2.5 g gelatin, 1.5 g sodium acetate and 2.4 g threonine in 1000 ml water, adjusted to pH 7.0 by dropping 1 N aqueous sodium hydroxide), 100 ml, was introduced into a flask and sterilized. To the medium thus prepared was inoculated 0.3 ml culture of known Corynebacterium pseudodiphteriticum subspecies ST-10 (Accession No. FERM P-13150) described in JP-A 10-94399 etc., previously cultured in a liquid medium having the above composition, and the microorganism was cultured under shaking at 30°C for 10 hours.

After the culture, the resulting culture was centrifuged (15000×g, 15 minutes, 4°C) to recover the microorganism. The recovered microorganism was suspended in 30 ml of 50 mM monopotassium phosphate-dipotassium phosphate buffer (pH 7.0), and this suspension was centrifuged (15000×g, 15 minutes, 4°C), whereby the washed microorganism was obtained.

The washed microorganism thus obtained was used to prepare chromosomal DNA by a method of J. C. Wang et al. (Appl. Microbiol. Biotechnol. 52:386-392 (1999)).

### (2-2) Preparation of a plasmid containing the present reductase gene (construction of plasmid pTrcPAR)

PCR is conducted in which an oligonucleotide having the nucleotide sequence of SEQ ID NO:5 and an oligonucleotide having the nucleotide sequence of SEQ ID NO:6 are used as the primers, and the chromosomal DNA prepared in the above item (2-1) is used as the template, and the following composition of the reaction solution and reactions conditions are used. (Expand High Fidelity PCR system manufactured by Roche Diagnostic was used.) Composition of the reaction solution

| | |
|---|---|
| Chromosomal DNA | 1 µl |
| dNTP (2.5 mM mix each) | 0.4 µl |
| Primers (20 pmol/µl) | 0 . 75 µl each |
| 10×Buffer (with MgCl₂) | 5 µl |
| enz. expand HiFi (3.5×10³ U/ml) | 0.375 µl |
| Ultra-purified water | 41.725 µl |

### Reaction conditions

A vessel containing the reaction solution having the above composition is set in PERKIN ELMER-GeneAmp PCR System 2400 and heated at 97°C (2 minutes), and the reaction solution is subjected to 10 cycles each consisting of reactions at 97°C (0.25 min)-55°C (0.5 min)-72°C (1.5 min) and then 20 cycles each consisting of reactions at 97°C (0.25 min)-55°C (0.5 min)-72°C (2.5 min), and the reaction solution is further kept at 72°C for 7 minutes.

To the PCR-amplified DNA fragment obtained by purifying the PCR reaction solution are added 2 restriction enzymes (NcoI and BamHI), and the DNA fragment is digested with the 2 enzymes. Then, the resulting DNA fragment is purified.

Separately, 2 restriction enzymes (NcoI and BamHI) are added to vector pTrc99A (Pharmacia), and the vector is digested with the 2 enzymes. Then, the resulting DNA fragment is purified.

The two purified DNA fragments thus obtained are mixed and ligated by T4 DNA ligase. The resulting ligation solution is transformed into E. coli DH5α.

From the resulting transformant, a plasmid containing the present reductase gene (also referred to hereinafter as plasmid pTrcPAR) is removed by QIAprep Spin Miniprep Kit (Qiagen).

### Example 3 (Preparation of the present reductase gene (No. 3))

### (3-1) Preparation of cDNA library

A medium (24 g/L potato dextrose broth (Becton Dickinson) in water), 100 ml, was introduced into a 500-ml flask and sterilized at 121°C for 15 minutes. To the medium thus prepared was inoculated 0.5 ml culture of Penicillium citrinum IFO4631 (available from Institute for Fermentation, Osaka (IFO), Japan (www.ifo.or.jp)) previously cultured (under shaking at 30°C for 48 hours) in a medium having the same composition, and the microorganism was cultured under shaking at 30°C for 72 hours. Thereafter, the resulting culture was centrifuged (8000×g, 10 minutes), and the formed pellet was recovered. The pellet was washed 3 times with 50 ml of 20 mM potassium phosphate buffer (pH 7.0), to give about 1.0 g washed microorganism.

The washed microorganism thus obtained was used to prepare the whole RNA by the guanidine thiocyanate phenol chloroform method. From the prepared whole RNA, RNA having poly(A) was obtained by using Oligotex(dT)30-Super(Takara Shuzo Co.,Ltd.).

A cDNA library was prepared according to the Gubler and Hoffman method. First, single-strand cDNA was prepared using the RNA having poly(A) obtained above, Oligo(dT)18-linker primers ((containing an XhoI site) manufactured by Takara Shuzo Co., Ltd.), RAV-2 Rtase and SuperScript II Rtase. To (the reaction solution containing) the prepared single-strand cDNA were added E. coli DNA polymerase, E. coli Rnase/E. coli DNA Ligase Mixture and T4 DNA polymerase, whereby double-strand cDNA was synthesized and blunt-ended.

The double-strand cDNA thus obtained was ligated with an EcoRI-NotI-BamHI adaptor (Takara Shuzo Co., Ltd.). After ligation, the resulting DNA was phosphorylated, cleaved with XhoI, applied to a spin column (Takara Shuzo Co., Ltd.) to remove low-molecular DNA, ligated with λZapII (cleaved with EcoRI-XhoI), and packaged by an in vitro packaging kit (Stratagene), whereby a cDNA library (also referred to hereinafter as cDNA library (A)) was prepared.

### (3-2) Preparation of a plasmid containing the present reductase gene (construction of plasmid pTrcRPc)

PCR was conducted in which an oligonucleotide having the nucleotide sequence of SEQ ID NO:7 and an oligonucleotide having the nucleotide sequence of SEQ ID NO:8 were used as the primers, and the cDNA library prepared in the above item (3-1) was used as the template, and the following composition of the reaction solution and reactions conditions were used. (Expand High Fidelity PCR system manufactured by Roche Diagnostic was used.)

### Composition of the reaction solution

| | |
|---|---|
| Stock solution of the cDNA library | 1 µl |
| dNTP (2.5 mM mix each) | 0.4 µl |
| Primers,(20 pmol/µl) | 0.75 µl each |
| 10×Buffer (with MgCl₂) | 5 µl |
| enz. expand HiFi (3.5×10³ U/ml) | 0.375 µl |
| Ultra-purified water | 41.725 µl |

### Reaction conditions

A vessel containing the reaction solution having the above composition was set in PERKIN ELMER-GeneAmp PCR System 2400 and heated at 97°C (2 minutes), and the reaction solution was subjected to 10 cycles each consisting of reactions at 97°C (0.25 min)-55°C (0.5 min)-72°C (1.5 min) and then 20 cycles each consisting of reactions at 97°C (0.25 min)-55°C (0.5 min)-72°C (2.5 min), and the reaction solution was further kept at 72°C for 7 minutes.

To the PCR-amplified DNA fragment obtained by purifying the PCR reaction solution were added 2 restriction enzymes (NcoI and BamHI), and the DNA fragment was digested with the 2 enzymes. Then, the resulting DNA fragment was purified.

Separately, 2 restriction enzymes (NcoI and BamHI) were added to vector pTrc99A (Pharmacia), and the vector was digested with the 2 enzymes. Then, the resulting DNA fragment was purified.

The two purified DNA fragments thus obtained were mixed and ligated by T4 DNA ligase. The resulting ligation solution was transformed into E. coli DH5α.

From the resulting transformant, a plasmid containing the present reductase gene (also referred to hereinafter as plasmid pTrcRPc) was removed by QIAprep Spin Miniprep Kit (Qiagen).

### Example 4 (Preparation of the present coenzyme regenerating enzyme gene)

(4-1) Preliminary procedure for preparation of a gene having a nucleotide sequence encoding the amino acid sequence of an enzyme having an ability to convert oxidized β-nicotinamide adenine dinucleotide etc. into reduced ones

An LB medium (1% trypton, 0.5% yeast extract, 1% sodium chloride), 100 ml, was introduced into a flask and sterilized. To the medium thus prepared was inoculated 0.3 ml culture of Bacillus megaterium IFO12108 (available from Institute for Fermentation, Osaka (IFO) (www.ifo.or.jp)) previously cultured in a liquid medium having the above composition, and the microorganism was cultured under shaking at 30°C for 10 hours.

After the culture, the resulting culture was centrifuged (15000×g, 15 minutes, 4°C) to recover the microorganism. The recovered microorganism was suspended in 30 ml of 50 mM monopotassium phosphate-dipotassium phosphate buffer (pH 7.0), and this suspension was centrifuged (15000×g, 15 minutes, 4°C), whereby a washed microorganism was obtained.

From the washed microorganism thus obtained, chromosomal DNA was purified by using Qiagen Genomic Tip (Qiagen) according to a method described in its attached manual.
(4-2) Preparation of a gene having a nucleotide sequence encoding the amino acid sequence of an enzyme having an ability to convert oxidized β-nicotinamide adenine dinucleotide etc. into reduced ones (No. 1: Construction of plasmid pSDGDH12)

An oligonucleotide having the nucleotide sequence of SEQ ID NO:9 and an oligonucleotide having the nucleotide sequence of SEQ ID NO:1.0 were synthesized on the basis of the amino acid sequence of glucose dehydrogenase derived from known Bacillus megaterium IWG3 described in The Journal of Biological Chemistry Vol. 264, No. 11, 6381-6385 (1989).

PCR was conducted in which the oligonucleotide having the nucleotide sequence of SEQ ID NO:9 and the oligonucleotide having the nucleotide sequence of SEQ ID NO:10 were used as the primers, and the chromosomal DNA purified in the above item (4-1) was used as the template, and the composition of the reaction solution and reactions conditions described in Example 2 (2-2) were used. (Expand High Fidelity PCR systemmanufactured by Roche Diagnostic was used.)

The PCR-amplified DNA fragment obtained by purifying the PCR reaction solution was ligated into an existing "PCR Product Insertion Site" in vector pCR2.1-TOPO by using a TOPO™ TA cloning kit manufactured by Invitrogen. The resulting ligation solution was transformed into E. coli DH5α.

From the resulting transformant, a plasmid containing a glucose dehydrogenase gene (also referred to hereinafter as plasmid pSDGDH12) was removed by QIAprep Spin Miniprep Kit (Qiagen)

Then, the removed plasmid pSDGDH12 was subjected as the template to sequencing reaction with Dye Terminator Cycle sequencing FS ready Reaction Kit (Perkin-Elmer), and then the nucleotide sequence of the resulting DNA was analyzed with a DNA sequencer 373A (Perkin-Elmer). The results are shown in SEQ ID NO:11.
(4-3) Preparation of a gene having a nucleotide sequence encoding the amino acid sequence of an enzyme having an ability to convert oxidized β-nicotinamide adenine dinucleotide phosphate etc. into reduced ones (No. 2: Construction of plasmid pAGDH12) (4-3-1) Construction of plasmid pTGDH12

An oligonucleotide having the nucleotide sequence of SEQ ID NO:13 and an oligonucleotide having the nucleotide sequence of SEQ ID NO:14 were synthesized on the basis of the nucleotide sequence of SEQ ID NO:11.

PCR was conducted in which the oligonucleotide having the nucleotide sequence of SEQ ID NO:13 and the oligonucleotide having the nucleotide sequence of SEQ ID NO:14 were used as the primers, and the chromosomal DNA prepared in the above item (4-1) was used as the template, and the following composition of the reaction solution and reactions conditions were used. (Expand High Fidelity PCR system manufactured by Roche Diagnostic was used.)

### Composition of the reaction solution

| | |
|---|---|
| Sock solution of chromosomal DNA | 1 µl |
| dNTP (2.5 mM mix each) | 0.4 µl |
| Primers (20 pmol/µl) | 0.75 µl each |
| 10×Buffer (with MgCl₂) | 5 µl |
| enz. expand HiFi (3.5×10³ U/ml) | 0.375 µl |
| Ultra-purified water | 41.725 µl |

### PCR reaction conditions

A vessel containing the reaction solution having the above composition was set in PERKIN ELMER-GeneAmp PCR System 2400 and heated at 97°C (2 minutes), and the reaction solution was subjected to 10 cycles each consisting of reactions at 97°C (0.25 min)-55°C (0.5 min)-72°C (1.5 min) and then to 20 cycles each consisting of reactions at 97°C (0.25 min)-55°C (0.5 min)-72°C (2.5 min), and the reaction solution was further kept at 72°C for 7 minutes.

To the PCR-amplified DNA fragment obtained by purifying the PCR reaction solution were added 2 restriction enzymes (NcoI and BamHI), and the DNA fragment was digested with the 2 enzymes. Then, the resulting DNA fragment was purified.

Separately, 2 restriction enzymes (NcoI and BamHI) were added to vector pTV118N (Takara Shuzo Co., Ltd.), and the vector was digested with the 2 enzymes. Then, the resulting DNA fragment was purified.

The two purified DNA fragments thus obtained were mixed and ligated by T4 DNA ligase. The resulting ligation solution was transformed into E. coli DH5α.

From the resulting transformant, a plasmid containing the present reductase gene (also referred to hereinafter as plasmid pTGDH12) was removed by QIAprep Spin Miniprep Kit (Qiagen).

### (4-3-2) Construction of plasmid pCGDH12

An oligonucleotide having the nucleotide sequence of SEQ ID NO:15 was synthesized on the basis of the nucleotide sequence of vector pTV118N (Takara Shuzo Co., Ltd.).

PCR was conducted in which the oligonucleotide having the nucleotide sequence of SEQ ID NO: 15 and an oligonucleotide having the nucleotide sequence of SEQ ID NO: 14 were used as the primers, and plasmid pTGDH12 was used as the template, and the following composition of the reaction solution and reactions conditions were used. (Expand High Fidelity PCR system manufactured by Roche Diagnostic was used.)

### Composition of the reaction solution

| | |
|---|---|
| Plasmid pTGDH12 solution | 1 µl |
| dNTP (2.5 mM mix each) | 0.4 µl |
| Primers (20 pmol/µl) | 0.75 µl each |
| 10×Buffer (with MgCl₂) | 5 µl |
| enz. expand HiFi (3.5×10³ U/ml) | 0.375 µl |
| Ultra-purified water | 41.725 µl |

### PCR reaction conditions

A vessel containing the reaction solution having the above composition was set in PERKIN ELMER-GeneAmp PCR System 2400 and heated at 97°C (2 minutes), and the reaction solution was subjected to 10 cycles each consisting of reactions at 97°C (0.25 min)-55°C (0.5 min)-72°C (1.5 min) and then 20 cycles each consisting of reactions at 97°C (0.25 min)-55°C (0.5 min)-72°C (2.5 min), and the reaction solution was further kept at 72°C for 7 minutes.

Using the resulting PCR reaction solution and TOPO™ TA cloning kit Ver. E, the about 1000-bp DNA fragment obtained by PCR was ligated into an existing "PCR Product Insertion Site" in vector pCR2.1-TOPO, and the ligation solution was transformed into *E. coli* DH5α.

From the resulting transformant , a plasmid containing the present reductase gene (also referred to hereinafter as plasmid pCGDH12) was removed by QIAprep Spin Miniprep Kit (Qiagen).

### (4-3-3) Construction of plasmid pAGDH12

By adding a restriction enzyme (BamHI) to plasmid pCGDH12, the plasmid was digested. Then, the resulting DNA fragment (about 1000 bp) was purified.

Separately, a restriction enzyme (BamHI) was added to vector pACYC184 (Nippon Gene) thereby digesting the plasmid. Then, the resulting DNA fragment was purified. Further, the DNA fragment was dephosphorylated with alkaline phosphatase (Takara Shuzo Co., Ltd.) in order to prevent self-ligation.

The two purified DNA fragments thus obtained were mixed and ligated by T4 DNA ligase. The resulting ligation solution was transformed into E. coli DH5α.

The resulting transformant was cultured in an LB agar medium containing 20 µg/ml chloramphenicol, and 4 colonies were selected at random from the proliferated colonies. Each of the selected colonies was inoculated into a sterilized LB medium (2 ml) containing 20 µg/ml chloramphenicol and cultured under shaking in a test tube (30°C, 24 hours). From the respective cultured microorganisms, plasmids were removed by QIAprep Spin Miniprep Kit (Qiagen). A part of each of the removed plasmids was digested with BamHI, and then the digest was electrophoresed, whereby it was confirmed that all the removed plasmids had the above DNA fragment (about 1000 bp) inserted therein.
(Hereinafter, the removed plasmids are also referred to as plasmid pAGDH12.)

### Example 5 (Preparation of a plasmid containing the reductase gene and the coenzyme regenerating enzyme gene (No. 1): Construction of plasmid pTrcPARSbG)

Two restriction enzymes (BamHI and XbaI) are added to plasmid pSDGDH12 prepared in Example 4 (4-2), and the plasmid is digested with the two enzymes. Then, the digested DNA fragment is purified.

Separately, two restriction enzymes (BamHI and XbaI) are added to plasmid pTrcPAR prepared in Example 2, and the plasmid is digested with the two enzymes. Then, the resulting DNA fragment is purified.

The two purified DNA fragments thus obtained are mixed and ligated by T4 DNA ligase. The resulting ligation solution is transformed into E. coli DH5α.

From the resulting transformant, a plasmid containing the reductase gene and the coenzyme regenerating gene (also referred to hereinafter as plasmid pTrcPARSbG) is removed by QIAprep Spin Miniprep Kit (Qiagen).

### Example 6 (Preparation of a plasmid containing the reductase gene and the coenzyme regenerating enzyme gene (No. 2): Construction of plasmid pTrcRSbG12)

Two restriction enzymes (BamHI and XbaI) were added to plasmid pSDGDH12 prepared in Example 4 (4-2), to digest the plasmid with the two enzymes. Then, the digested DNA fragment was purified.

Separately, two restriction enzymes (BamHI and XbaI) were added to plasmid pTrcRPc prepared in Example 3, to digest the plasmid with the two enzymes. Then, the digested DNA was purified.

The two purified DNA fragments thus obtained were mixed and ligated by T4 DNA ligase. The resulting ligation solution was transformed into E. coli DH5α.

From the resulting transformant, a plasmid containing the reductase gene and the coenzyme-regenerating gene (also referred to hereinafter as plasmid pTrcRSbG12) was removed by QIAprep Spin Miniprep Kit (Qiagen).

### Example 7. Preparation of a transformant containing the reductase gene and the coenzyme regenerating enzyme gene (No. 1)

The plasmid pTrcPARSbG prepared in Example 5 is used to transform *E. coli* HB101. The resulting transformant is inoculated into a sterilized LB medium (100 ml×3 tubes) containing 0.4 mM IPTG, 0.01% (w/v) ZnCl₂ and 50 µg/ml ampicillin, and then cultured under shaking (30°C, 18 hours). After the culture, the culture solution is centrifuged and washed, to recover the washed microorganism.

### Example 8 (Preparation of a transformant containing the reductase gene and the coenzyme regenerating enzyme gene (No. 2))

The plasmid pTrcRSbG12 prepared in Example 6 was used to transform E. coli HB101. The resulting transformant was inoculated into a sterilized LB medium (100 ml×3 tubes) containing 0.1 mM IPTG and 50 µg/ml ampicillin, and then cultured under shaking (30°C, 18 hours). After the culture, the culture solution was centrifuged and washed, to recover 1.2 g washed microorganism.

### Example 9 Preparation of a transformant containing the reductase gene and the coenzyme regenerating enzyme gene (No. 3)

The plasmid pTrcRPc prepared in Example 3 and the plasmid pAGDH12 prepared in Example 4 (4-3) are used to transform E. coli HB101. The resulting transformant is inoculated into a sterilized LB medium (100 ml×3 tubes) containing 0.4 mM IPTG, 20 µg/ml chloramphenicol and 50 µg/ml ampicillin, and then cultured under shaking (30°C, 18 hours). After the culture, the culture solution is centrifuged and washed, to recover the washed microorganism.

### Example 10 Process for producing 3-hydroxycyclohexanone

The washed microorganism, 15 mg, prepared in Example 1, 2.5 mg NAD⁺ (reagent from Oriental Yeast Co., Ltd.) and 5% (v/v) 2-propanol were added to 2.0 ml of 100 mM monopotassium phosphate-dipotassium phosphate buffer (pH 6.5). To this mixture was added 50 mg of 1,3-cyclohexanedione (reagent from Aldrich). The mixture (reaction solution) thus obtained was allowed to react under stirring at 30°C for 21 hours. After the reaction was finished, 2.0 ml butyl acetate was poured into the reaction solution, then stirred and centrifuged, whereby the organic layer and the aqueous layer were recovered respectively. By GC analysis, formation of 3-hydroxycyclohexanone was confirmed. The yield was 11% (GC analysis).

### GC conditions

| | |
|---|---|
| Column: | DB-1 (0.53 mmφ×30 m, 1.5 µm) |
| Injection port temperature: | 170°C |
| Detector (FID): | 300°C |
| Column chamber temperature: | 70°C (kept for 5 minutes) |
| →5°C/min .→ 170°C (kept for 0 minute) → (increasing temperature: 30°C/min.) → 290°C | |
| Carrier gas: | 10 mL/min. |
| Retention time of 3-hydroxycyclohexanone: | 9.2 minutes |

3-Hydroxycyclohexanone was prepared by a known method (Journal of Organic Chemistry, p. 1046 (2001)).

### Example 12 (Process for producing 3-hydroxycyclohexanone (No. 2))

10 mg commercial enzyme KRED1004 (Biocatalytics, US), 25 mg NADP⁺ (reagent from Oriental Yeast), 1.0 g glucose and a commercial enzyme glucose dehydrogenase (Amano Pharmaceutical Co., Ltd.) were added to 25 g of 100 mM monopotassium phosphate-dipotassium phosphate buffer (pH 6.5). After 50 mg of 1,3-cyclohexanedione was added to this mixture, the mixture was allowed to react under stirring at 30°C for 4 hours. During the reaction, the reaction solution was kept at pH 6.4 to 6.5 by suitably adding 15% aqueous sodium carbonate. After the reaction was finished, 25 ml ethyl acetate was poured into the reaction solution, then stirred and centrifuged, whereby the organic layer and the aqueous layer were recovered respectively. 25 ml ethyl acetate was added again to the recovered aqueous layer, and the same procedure was conducted twice. The organic layers thus obtained were combined, concentrated, dissolved in 30 ml chloroform and dried over anhydrous Na₂SO₄. After drying, chloroform was distilled away, whereby 3-hydroxycyclohexanone (20 mg) having the following physical properties was obtained. H-NMR (δ, ppm, CDCl₃); 1.8 (2H, m), 2.0 (2H, m), 2.4 (2H, m), 2.7 (2H, m), 4.2 (1H, m)
[α]D = -18° (c = 0.25, CDCl₃)

### Reference Example 1 (Acquisition of a microorganism having an ability to reduce 1,3-cyclohexanedione to form 3-hydroxycyclohexanone)

### (1-1) Preparation of a washed microorganism

A commercial microorganism or a microorganism isolated from soil etc. is inoculated into a sterilized LB medium (10 ml) and cultured under shaking (30°C, 18 hours). After the culture, the culture solution is centrifuged and washed, to recover the washed microorganism.

### (1-2) Screening

The washed microorganism, 1 g, prepared in the above item (1-1), 12 mg NADP⁺, 12 mg NAD⁺ and 2.5 g glucose are added to 20 ml of 100 mM monopotassium phosphate-dipotassium phosphate buffer (pH 6.5). After 240 mg of 1,3-cyclohexanedione is added to this mixture, the pH value of this mixture is adjusted to 6.5 with 15% aqueous sodium carbonate. The mixture (reaction solution) thus obtained is allowed to react under stirring at 30°C for 4 hours. After the reaction is finished, 25 ml ethyl acetate is poured into the reaction solution, then stirred and centrifuged, whereby the organic layer and the aqueous layer are recovered respectively. 25 ml ethyl acetate was added again to the recovered aqueous layer, and the same procedure is conducted. The organic layers thus obtained are combined, concentrated, dissolved in 30 ml chloroform and dried over anhydrous Na₂SO₄. After drying, chloroform is distilled away to give residues. By qualitative and/or quantitative analysis using liquid chromatography or gas chromatography and measurement of optical rotation, it is confirmed that 3-hydroxycyclohexanone was contained in the resulting residues.

As described above, 3-hydroxycyclohexanone can be produced efficiently or readily according to the invention.

### Sequence Listing Free Text

SEQ ID NO:5 shows an oligonucleotide which is a primer designed for PCR.
SEQ ID NO:6 shows an oligonucleotide which is a primer designed for PCR.
SEQ ID NO:7 shows an oligonucleotide which is a primer designed for PCR.
SEQ ID NO:8 shows an oligonucleotide which is a primer designed for PCR.
SEQ ID NO:9 shows an oligonucleotide which is a primer designed for PCR.
SEQ ID NO:10 shows an oligonucleotide which is a primer designed for PCR.
SEQ ID NO:13 shows an oligonucleotide which is a primer designed for PCR.
SEQ ID NO:14 shows an oligonucleotide which is a primer designed for PCR.
SEQ ID NO:15 shows an oligonucleotide which is a primer designed for PCR.

## Claims

1. A process for producing optically active 3-hydroxycyclohexanone, which comprises the steps of:
(a) reacting 1,3-cyclohexanedione with
(i) an enzyme having an ability to reduce 1,3-cyclohexanedione to 3-hydroxycyclohexanone,
(ii) a microorganism producing the enzyme defined in (i), or
(iii) a treated material of the enzyme as defined in (i) or of the microorganism defined in (ii), and
(b) recovering resulting 3-hydroxycyclohexanone, wherein:
i) said enzyme having the ability to reduce 1,3-cyclohexanedione to 3-hydroxycyclohexanone is an enzyme:
derived from a microorganism belonging to the genus *Arthrobacter, Rhodotorula, Bacillus, Pseudomonas, Streptomyces, Candida, Corynebacterium* or *Penicillium;* or
having any one of the following amino acid sequences:
the amino acid sequence set forth in SEQ ID NO:1 or 3, an amino acid sequence comprising deletion, substitution, or addition of one or more amino acids in the amino acid sequence of SEQ ID NO:1 or 3, an amino acid sequence encoded by the nucleotide sequence set forth in SEQ ID NO:2 or 4, and an amino acid sequence encoded by a nucleotide sequence of a DNA having 90% or more sequence identity with DNA of the nucleotide sequence of SEQ ID NO:2 or 4; or
ii) said microorganism producing the enzyme is a transformant having an introduced plasmid containing:
a nucleotide sequence of the nucleotide sequence of SEQ ID NO:2 or 4, or a nucleotide sequence of a DNA having 90% or more sequence identity with DNA of the nucleotide sequence of SEQ ID NO:2 or 4; or
iii) said treated material of the microorganism producing the enzyme having an ability to reduce 1,3-cyclohexanedione to 3-hydroxycyclohexanone is dead microbial cells of the microorganism as defined in ii) above.

2. A process according to claim 1, wherein the transformant is *Escherichia coli.*

3. A process according to claim 1, wherein the enzyme has the amino acid sequence as set forth in SEQ ID NO:1.

4. A process according to claim 1, wherein the enzyme has the amino acid sequence as set forth in SEQ ID NO:3.

5. A process according to claim 1, wherein the enzyme has an amino acid sequence encoded by the nucleotide sequence as set forth in SEQ ID NO:2.

6. A process according to claim 1, wherein the enzyme has an amino acid sequence encoded by the nucleotide sequence as set forth in SEQ ID NO:4.

7. A process according to claim 1, wherein the transformant is a transformant produced by introducing:
the plasmid containing
a nucleotide sequence encoding the enzyme as defined in ii) and optionally
a plasmid containing a nucleotide sequence that encodes an enzyme that regenerates a coenzyme on which the enzyme having the ability to reduce 1,3-cyclohexanedione to 3-hydroxycyclohexanone depends.

8. A process according to claim 7, wherein the coenzyme is NADH/NAD⁺ (nicotinamide adenine dinucleotide) or NADPH/NADP⁺ (nicotinamide adenine dinucleotide phosphate).

9. A process according to claim 1, wherein 1, 3-cyclohexanedione is allowed to react with the transformant or dead microbial cells thereof in the presence of a fatty alcohol.

10. A process according to claim 9, wherein the fatty alcohol is an alcohol having a boiling point of 200°C or less.

11. A process according to claim 9, wherein the fatty alcohol is 2-propanol.

12. A process according to claim 1, wherein 1, 3-cyclohexanedione is allowed to react with the transformant or dead microbial cells thereof in the presence of glucose.

13. Optically active 3-hydroxycyclohexanone.

## Revendications

1. Procédé de production de 3-hydroxycyclohexanone optiquement active, qui comprend les étapes de :
(a) réaction de 1,3-cyclohexanedione avec
(i) une enzyme possédant la capacité de réduire la 1,3-cyclohexanedione en 3-hydroxycyclohexanone,
(ii) un micro-organisme produisant l'enzyme définie au (i), ou
(iii) un matériau traité de l'enzyme telle que définie au (i) ou du micro-organisme défini au (ii), et
(b) récupération de la 3-hydroxycyclohexanone résultante,
dans lequel :
i) ladite enzyme possédant la capacité de réduire la 1,3-cyclohexanedione en 3-hydroxycyclohexanone est une enzyme :
dérivée d'un micro-organisme appartenant au genre *Arthrobacter, Rhodotorula, Bacillus, Pseudomonas, Streptomyces, Candida, Corynebacterium* ou *Penicillium ;* ou
possédant l'une quelconque des séquences d'acides aminés suivantes :
la séquence d'acides aminés représentée par SEQ ID N° : 1 ou 3, une séquence d'acides aminés comprenant une délétion, une substitution ou une addition d'un ou plusieurs acides aminés dans la séquence d'acides aminés de SEQ ID N° : 1 ou 3, une séquence d'acides aminés codée par la séquence nucléotidique représentée par SEQ ID N° : 2 ou 4, et une séquence d'acides aminés codée par une séquence nucléotidique d'un ADN possédant une identité de séquence de 90 % ou plus avec l'ADN de la séquence nucléotidique de SEQ ID N° : 2 ou 4 ; ou
ii) ledit micro-organisme produisant l'enzyme est un transformant comportant un plasmide introduit contenant :
une séquence nucléotidique de la séquence nucléotidique de SEQ ID N° : 2 ou 4, ou une séquence nucléotidique d'un ADN possédant une identité de séquence de 90 % ou plus avec l'ADN de la séquence nucléotidique de SEQ ID N° : 2 ou 4 ; ou
iii) ledit matériau traité du micro-organisme produisant l'enzyme possédant la capacité de réduire la 1,3-cyclohexanedione en 3-hydroxycyclohexanone consiste en des cellules microbiennes mortes du micro-organisme tel que défini au (ii) ci-dessus.

2. Procédé selon la revendication 1, dans lequel le transformant est *Escherichia coli.*

3. Procédé selon la revendication 1, dans lequel l'enzyme possède la séquence d'acides aminés telle que représentée par SEQ ID N° : 1.

4. Procédé selon la revendication 1, dans lequel l'enzyme possède la séquence d'acides aminés telle que représentée par SEQ ID N° : 3.

5. Procédé selon la revendication 1, dans lequel l'enzyme possède une séquence d'acides aminés codée par la séquence nucléotidique telle que représentée par SEQ ID N° : 2.

6. Procédé selon la revendication 1, dans lequel l'enzyme possède une séquence d'acides aminés codée par la séquence nucléotidique telle que représentée par SEQ ID N° : 4.

7. Procédé selon la revendication 1, dans lequel le transformant est un transformant produit en introduisant :
le plasmide contenant :
une séquence nucléotidique codant pour l'enzyme telle que définie au (ii) et éventuellement
un plasmide contenant une séquence nucléotidique qui code pour une enzyme qui régénère une coenzyme dont dépend l'enzyme possédant la capacité de réduire la 1,3-cyclohexanedione en 3-hydroxycyclohexanone.

8. Procédé selon la revendication 7, dans lequel la coenzyme est le NADH/NAD⁺ (nicotinamide adénine dinucléotide) ou le NADPH/NADP⁺ (nicotinamide adénine dinucléotide phosphate).

9. Procédé selon la revendication 1, dans lequel la 1,3-cyclohexanedione est mise à réagir avec le transformant ou des cellules microbiennes mortes de celui-ci en présence d'un alcool gras.

10. Procédé selon la revendication 9, dans lequel l'alcool gras est un alcool possédant un point d'ébullition de 200°C ou moins.

11. Procédé selon la revendication 9, dans lequel l'alcool gras est le 2-propanol.

12. Procédé selon la revendication 1, dans lequel la 1,3-cyclohexanedione est mise à réagir avec le transformant ou des cellules microbiennes mortes de celui-ci en présence de glucose.

13. 3-Hydroxycyclohexanone optiquement active.

## Patentansprüche

1. Verfahren zur Herstellung von optisch aktivem 3-Hydroxycyclohexanon mit den Schritten:
(a) Reagieren von 1,3-Cyclohexandion mit
(I) einem Enzym mit der Fähigkeit, 1,3-Cyclohexandion zu 3-Hydroxycyclohexanon zu reduzieren,
(II) einem Mikroorganismus, der das in (I) definierte Enzym produziert, oder
(III) einem behandelten Material aus dem in (I) definierten Enzym oder dem in (II) definierten Mikroorganismus; und
(b) Rückgewinnen des resultierenden 3-Hydroxycyclohexanons,
**dadurch gekennzeichnet, dass**
I) das Enzym mit der Fähigkeit, 1,3-Cyclohexandion zu 3-Hydroxycyclohexanon zu reduzieren, ein Enzym ist,
das von einem Mikroorganismus abgeleitet ist, der zu der Gattung *Arthrobacter, Rhodotorula, Bacillus, Pseudomonas, Streptomyces, Candida, Corynebacterium* oder *Penicillium* gehört, oder
das eine der folgenden Aminosäuresequenzen hat: die bei SEQ ID NO: 1 oder 3 angegebene Aminosäuresequenz, eine Aminosäuresequenz mit einer Deletion, Substitution oder Addition einer oder mehrerer Aminosäuren in der Aminosäuresequenz der SEQ ID NO: 1 oder 3, eine Aminosäuresequenz, die mit der bei SEQ ID NO: 2 oder 4 angegebenen Nucleotidsequenz codiert ist, und eine Aminosäuresequenz, die mit einer Nucleotidsequenz einer DNA codiert ist, die eine Sequenz-Identität von 90 % oder mehr mit der DNA der Nucleotidsequenz der SEQ ID NO: 2 oder 4 hat, oder
II) der Mikroorganismus, der das Enzym produziert, eine Transformante mit einem integrierten Plasmid ist, das eine Nucleotidsequenz der Nucleotidsequenz der SEQ ID NO: 2 oder 4 oder eine Nucleotidsequenz einer DNA mit einer Sequenz-Identität von 90 % oder mehr mit der DNA der Nucleotidsequenz der SEQ ID NO: 2 oder 4 enthält, oder
III) das behandelte Material aus dem Mikroorganismus, der das Enzym mit der Fähigkeit, 1,3-Cyclohexandion zu 3-Hydroxycyclohexanon zu reduzieren, produziert, tote Mikrobenzellen des unter II) definierten Mikroorganismus umfasst.

2. Verfahren nach Anspruch 1, **dadurch gekennzeichnet, dass** die Transformante *Escherichia coli* ist.

3. Verfahren nach Anspruch 1, **dadurch gekennzeichnet, dass** das Enzym die bei SEQ ID NO: 1 angegebene Aminosäuresequenz hat.

4. Verfahren nach Anspruch 1, **dadurch gekennzeichnet, dass** das Enzym die bei SEQ ID NO: 3 angegebene Aminosäuresequenz hat.

5. Verfahren nach Anspruch 1, **dadurch gekennzeichnet, dass** das Enzym eine Aminosäuresequenz hat, die mit der bei SEQ ID NO: 2 angegebenen Nucleotidsequenz codiert ist.

6. Verfahren nach Anspruch 1, **dadurch gekennzeichnet, dass** das Enzym eine Aminosäuresequenz hat, die mit der bei SEQ ID NO: 4 angegebenen Nucleotidsequenz codiert ist.

7. Verfahren nach Anspruch 1, **dadurch gekennzeichnet, dass** die Transformante eine Transformante ist, die durch Integrieren
des Plasmids, das eine unter II) definierte Nucleotidsequenz, die das Enzym codiert, enthält, und wahlweise
eines Plasmids, das eine Nucleotidsequenz enthält, die ein Enzym codiert, das ein Coenzym regeneriert, von dem das Enzym mit der Fähigkeit, 1,3-Cyclohexandion zu 3-Hydroxycyclohexanon zu reduzieren, abhängt,
produziert wird.

8. Verfahren nach Anspruch 7, **dadurch gekennzeichnet, dass** das Coenzym NADH/NAD⁺ (Nicotinamid-adenin-dinucleotid) oder NADPH/NADP⁺ (Nicotinamid-adenindinucleotidphosphat) ist.

9. Verfahren nach Anspruch 1, **dadurch gekennzeichnet, dass** 1,3-Cyclohexandion mit der Transformanten oder deren toten Mikrobenzellen in Gegenwart eines Fettalkohols reagieren gelassen wird.

10. Verfahren nach Anspruch 9, **dadurch gekennzeichnet, dass** der Fettalkohol ein Alkohol mit einem Kochpunkt von 200 °C oder weniger ist.

11. Verfahren nach Anspruch 9, **dadurch gekennzeichnet, dass** der Fettalkohol 2-Propanol ist.

12. Verfahren nach Anspruch 1, **dadurch gekennzeichnet, dass** 1,3-Cyclohexandion mit der Transformanten oder deren toten Mikrobenzellen in Gegenwart von Glucose reagieren gelassen wird.

13. Optisch aktives 3-Hydroxycyclohexanon.
